# EUROPEAN PATENT APPLICATION

(11) **EP 4 631 426 A1**
(43) Date of publication of application: **15.10.2025**
(21) Application number: 25169362.8
(22) Date of filing: 09.04.2025
(51) Int. Cl.: A61B 5/055, G01R 33/44, G01R 33/485, G01R 33/56, G01R 33/00, G01R 33/62, G01R 33/34, G01R 33/3415, G01R 33/36

(54) **SYSTEM AND METHODS FOR ORGAN-TARGETED MULTINUCLEAR FUNCTIONAL AND MOLECULAR MAGNETIC RESONANCE IMAGING**

(30) Priority: 11.04.2024 US 202463632751 P
(71) Applicant: Lakehead University, Thunder Bay, ON P7B 5E1 (CA)
(72) Inventor: Albert, Mitchell, Thunder Bay, P7B 5E1 (CA); Shepelytskyi, Yurii, Thunder Bay, P7B 5E1 (CA); Batarchuk, Viktoriia, Thunder Bay, P7B 5E1 (CA); Reznik, Alla, Thunder Bay, P7B 5E1 (CA); Hodgson, Aaron, Thunder Bay, P7B 5E1 (CA)
(74) Representative: Franke, Dirk

(57) **Abstract**

Described herein is a molecular imaging system comprising two or more MRI coils or coil arrays, where at least one coil is tuned to ¹H resonance frequency and at least one coil is tuned to the resonance frequency of a nuclei such as ¹⁹F, ³¹P, ¹²⁹Xe, ¹³C or ²³Na. The imaging system may also contain passive frequency-selective flux focusing RF elements that are utilized to amplify the generated RF signal and, therefore, maximize the sensitivity and overall performance of the imaging system.

## Description

### PRIOR APPLICATION INFORMATION

The instant application claims the benefit of US Provisional Patent Application 63/632,751, filed April 11, 2024 and entitled "A SYSTEM AND METHODS FOR ORGAN-TARGETED MULTINUCLEAR FUNCTIONAL AND MOLECULAR MAGNETIC RESONANCE IMAGING", the entire contents of which are incorporated herein by reference for all purposes.

### BACKGROUND OF THE INVENTION

Although Magnetic Resonance Imaging (MRI) is widely used, MRI has the lowest sensitivity compared to other common imaging modalities.

Ideally, the sensitivity of MRI-based methods needs to be increased, for example, by signal enhancement, to allow for molecular imaging and/or metabolic imaging. For example, such an improved system would allow for higher resolution, a smaller field of view and the analysis of thinner slices.

### SUMMARY OF THE INVENTION

According to an aspect of the invention, there is provided a method of molecular imaging of a body region of an individual comprising:
administering a molecular imaging agent that contains one or more X-nuclei selected from the group consisting of ¹⁹F, ²³Na, ¹²⁹Xe, ³¹P and ¹³C, to an individual;
allowing the molecular imaging agent to distribute through the body of the individual, said imaging agent being metabolizable under certain conditions within the body of the individual;
acquiring an anatomical image of a body region of interest of the individual using a ¹H coil;
acquiring one or more X-nuclei image(s) of the body region of interest of the molecular imaging agent and any metabolites of the molecular imaging agent within the body region of the individual using an X-nuclei coil specific for at least one of the X-nuclei in the molecular imaging agent;
superimposing the one or more X-nuclei image(s) of the body region of interest on the anatomical image of the body region of interest, thereby localizing metabolic activity and biodistribution of the molecular imaging agent within the body region of interest.

As will be appreciated by one of skill in the art, the number of X-nuclei images acquired will heavily depend on the specific type of the metabolic imaging agent used. For example, in some embodiments, a single image may include both the metabolic imaging agent and any metabolites generated therefrom.

As will be appreciated by one of skill in the art, the ¹H RF coil may be a stand-alone coil as discussed herein or may be part of a pre-existing imaging system, that is, an imaging system that the individual is placed into and then the X-nuclei RF coil is placed over the body region of interest, as discussed herein.

According to an aspect of the invention, there is provided a method of molecular breast imaging of an individual comprising:
administering a molecular imaging agent that contains one or more X-nuclei selected from the group consisting of ¹⁹F, ²³Na, ¹²⁹Xe, ³¹P and ¹³C, into an individual;
allowing a molecular imaging agent to distribute through the body of the individual, said imaging agent being metabolizable under certain conditions within the body of the individual;
acquiring an anatomical breast image using a ¹H coil of a portion of the breast tissue of the individual;
acquiring one or more X-nuclei image(s) of the molecular imaging agent and any metabolites of the molecular imaging agent of the portion of the breast tissue of the individual using an X-nuclei coil specific for at least one of the X-nuclei in the molecular imaging agent; and
superimposing the one or more X-nuclei image(s) on the anatomical breast image, thereby localizing metabolic activity and biodistribution of the molecular imaging agent within the portion of the breast tissue of the individual.

As will be appreciated by one of skill in the art, the number of X-nuclei images acquired will heavily depend on the specific type of the metabolic imaging agent used. For example, in some embodiments, a single image may include both the metabolic imaging agent and any metabolites generated therefrom.

In some embodiments of the invention, the method is characterized in that since the ¹H RF coil and the X-nuclei RF coil are tuned to different frequencies, they can work simultaneously with no interference and can take images of the same body region simultaneously, that is, the ¹H RF coil and the X-nuclei RF-coil can be physically placed together over the body region of interest, thereby allowing for perfect alignment of the images.

As will be appreciated by one of skill in the art, the ¹H RF coil may be a stand-alone coil as discussed herein or may be part of a pre-existing imaging system, that is, an imaging system that the individual is placed into and then the X-nuclei RF coil is placed over the body region of interest, as discussed herein.

According to another aspect of the invention, there is provided an MRI imaging device comprising a ¹H MRI coil and at least one X-nuclei coil positioned on a support structure having a shape suitable for enclosing a body portion, for example, a tissue portion or an organ of interest, wherein the X-nuclei is selected from the group consisting of ¹⁹F, ²³Na, ¹²⁹Xe, ³¹P and ¹³C.

According to another aspect of the invention, there is provided a method of molecular abdominal or chest imaging of an individual comprising:
injecting a molecular imaging agent that comprises one or more X-nuclei selected from the group consisting of ¹⁹F, ²³Na, ¹²⁹Xe, ³¹P and ¹³C, into the individual;
allowing the molecular imaging agent to distribute through the body of the individual, said molecular imaging agent being metabolizable in the body of the individual under certain conditions;
acquiring an anatomical abdominal or chest image using a ¹H RF coil;
acquiring one or more X-nuclei image(s) of the molecular imaging agent of a portion of the individual's body corresponding to the anatomical abdominal or chest image and any metabolites of the molecular imaging agent using an X-nuclei coil specific for at least one of the X-nuclei in the molecular imaging agent;
superimposing the one or more X-nuclei image(s) on the anatomical abdominal or chest image, thereby localizing metabolic activity and biodistribution of the molecular imaging agent in the abdomen or chest of the individual.

As will be appreciated by one of skill in the art, the number of X-nuclei images acquired will heavily depend on the specific type of the metabolic imaging agent used. For example, in some embodiments, a single image may include both the metabolic imaging agent and any metabolites generated therefrom.

As will be appreciated by one of skill in the art, the ¹H RF coil may be a stand-alone coil as discussed herein or may be part of a pre-existing imaging system, that is, an imaging system that the individual is placed into and then the X-nuclei RF coil is placed over the body region of interest, as discussed herein.

According to another aspect of the invention, there is provided a method of molecular brain imaging of an individual comprising:
injecting a molecular imaging agent that comprises one or more X-nuclei selected from the group consisting of ¹⁹F, ²³Na, ¹²⁹Xe, ³¹P and ¹³C, into the individual;
allowing the molecular imaging agent to distribute throughout the body, said molecular imaging agent being metabolizable in the body under certain conditions;
acquiring an anatomical brain image using a ¹H RF coil;
acquiring one or more X-nuclei image(s) of the molecular imaging agent and any metabolites of the molecular imaging agent using an X-nuclei coil specific for at least one of the X-nuclei in the molecular imaging agent; and
superimposing the one or more X-nuclei image(s) on the anatomical brain image, thereby localizing metabolic activity and biodistribution of the molecular imaging agent in the brain of the individual.

As will be appreciated by one of skill in the art, the number of X-nuclei images acquired will heavily depend on the specific type of the metabolic imaging agent used. For example, in some embodiments, a single image may include both the metabolic imaging agent and any metabolites generated therefrom.

As will be appreciated by one of skill in the art, the ¹H RF coil may be a stand-alone coil as discussed herein or may be part of a pre-existing imaging system, that is, an imaging system that the individual is placed into and then the X-nuclei RF coil is placed over the body region of interest, as discussed herein.

As used herein, "certain conditions" refers to "certain metabolic conditions" within the body wherein the metabolic imaging agent will be reacted on and/or modified by the metabolism of the individual. Furthermore, "certain conditions" may also refer to the time period required for these metabolic reactions to take place. As such, "certain conditions" refers not only to the presence or absence of the metabolic conditions necessary for reaction of the metabolic imaging agent but also to the rate at which these metabolic reactions occur.

### BRIEF DESCRIPTION OF THE DRAWINGS

Figure 1. An example of the imaging coil system with one multinuclear coil (3) and one passive frequency-selective flux focusing element (5).
Figure 2. An example of the imaging coil system with two multinuclear coils (3,4) and one passive frequency selective flux-focusing element (8).
Figure 3. An example of the imaging coil system with one birdcage RF coil (2) and passive frequency-selective flux focusing element (3) placed on the inner surface of the RF coil rigid dielectric frame (1).
Figure 4. Some possible examples of a passive frequency-selective flux-focusing elements (Lenz resonators) that can be used as part of the invention.
Figure 5. ¹⁹F signals (A-D) acquired from the rat brain during ¹⁹F-labled glucose infusion. (E) shows images of glucose and its metabolites (bottom raw) as well as their main kinetic parameters (top raw).
Figure 6. Images of a pomegranate acquired using (A) conventional quadrature birdcage proton coil (PRIOR ART) and (B) our proposed imaging system with two passive flux-focusing resonators located inside the detection coil at 12 cm from the pomegranate. The resulting signal-to-noise ratio is twice larger compared to the conventional imaging system.

### DESCRIPTION OF THE PREFERRED EMBODIMENTS

Unless defined otherwise, all technical and scientific terms used herein have the same meaning as commonly understood by one of ordinary skill in the art to which the invention belongs. Although any methods and materials similar or equivalent to those described herein can be used in the practice or testing of the present invention, the preferred methods and materials are now described. All publications mentioned hereunder are incorporated herein by reference.

Described herein is an MRI imaging component and system incorporating the MRI imaging component that is capable of rapid and accurate detection of all clinically relevant metabolites and pharmacokinetics of most commonly used pharmaceutical agents which has not been achieved in the past with MRI. Our system renders the MRI as a molecular imaging modality that can satisfy the requirements of modern personalized medicine.

For example, in some embodiments, the imaging system is connected to an MRI scanner directly and is used as a signal receiver on its own, as discussed herein.

Specifically, molecular imaging has important implications for personalized medicine because it allows for non-invasive visualization of biological processes at the molecular and cellular level. This can provide valuable information about the location, activity, and metabolism of specific molecules or cells within an individual's body. Unfortunately, conventional MRI imaging is not currently capable of molecular imaging due to its low sensitivity, albeit its high resolution and lack of ionizing radiation make this imaging modality quite attractive for molecular imaging purposes.

The following description relates to various embodiments of the disclosed imaging system assembly and methods of using it to detect for example pharmaceutical drug metabolism and molecular biomarkers in both clinical and preclinical settings. Our unique system allows conduction of functional and molecular MRI imaging for at least the following applications: 1) drug metabolism; 2) biomarkers; 3) glucose metabolism for inflammation and cancer detection; 4) functional imaging with inhalation gas agents; and 5) pharmacokinetics imaging including chemical drugs, biosensors, and hormones.

In some embodiments, the imaging system contains multiple MRI coils or coil arrays, where at least one coil is tuned to ¹H resonance frequency and at least one coil is tuned to the resonance frequency of the other nuclei with a high gyromagnetic ratio or natural abundance. Examples of such nuclei are ¹⁹F, ³¹P, ¹²⁹Xe, ¹³C and ²³Na. As will be appreciated by one of skill in the art, these nuclei can produce sufficient MRI signal and, therefore, be traced and quantified using this medical imaging modality. In some embodiments, the system can have up to five X-nuclei coils. The imaging system may also contain passive frequency-selective flux focusing RF elements that are utilized to amplify the generated RF signal and, therefore, maximize the sensitivity and overall performance of the imaging system, as discussed below.

As will be appreciated by those of skill in the art, frequencies depend on the external magnetic field of the MRI scanner. For example:

| Nucleus | 1.5T | 3.0T |
|---|---|---|
| ¹H | 63.8 MHz | 127.6 MHz |
| ¹⁹F | 60.08 MHz | 120.15 MHz |
| ²³Na | 18.9 MHz | 33.79 MHz |
| ¹²⁹Xe | 17.67 MHz | 35.33 MHz |
| ³¹P | 25.8 MHz | 51.6 MHz |
| ¹³C | 16.06 MHz | 32.12 MHz |

As would be appreciated by one skilled in the art, the system may contain only ¹H MRI coil coupled a passive frequency-selective flux focusing RF elements for ¹H metabolic imaging.

Furthermore, while particular body parts, specifically, the breast, brain, chest and abdomen and organs, are provided as examples for molecular imaging, it is important to note that any body part and/or organ and/or limb and/or tissue portion may be analyzed by the molecular MRI imaging system, devices and/or methods of the invention.

According to some aspects of the invention, the system can be utilized either in an MRI scanner or in a PET/MRI scanner. As will be appreciated by one of skill in the art, in these embodiments, the ¹H RF coil is part of a pre-existing imaging system, that is, an imaging system that the individual is placed into and then the X-nuclei RF coil is placed over the body region of interest, as discussed herein.

As will be appreciated by one skilled in the art, the imaging system's shape and size can vary depending on the organ of interest being imaged. In some embodiments, the imaging system can be utilized for breast molecular, metabolic, and anatomical MRI imaging. As discussed herein, the system comprises an ¹H MRI coil and at least one X-nuclei MRI coil.

According to some aspects of the invention, the system can include multiple X-nuclei coils (up to five) that can be either mechanically or electrically activated or deactivated.

In some embodiments, these MRI coils may be positioned for example in an adjustable, bra-like support structure.

In some embodiments, an ¹H MRI coil comprises two coil arrays with multiple segments in each. The first coil array may be housed on a first cup-shaped support structure, whereas the second coil array may be housed on a second cup-shaped support structure. By varying the number of coil elements in each array, the distance between adjacent RF elements may be reduced and the coil may be positioned closer to the anatomy being imaged resulting in a higher signal-to-noise ratio (SNR).

The X-nuclei coil comprises two coil arrays each tuned to a resonance frequency of nuclei of interest (¹⁹F, ³¹P, ¹²⁹Xe, ²³Na).

In some embodiments, the first X-nuclei coil array may be housed inside a first cup-shaped support structure, whereas the second coil array may be housed inside a second cup-shaped support structure. In these embodiments, positioning the X-nuclei coil elements inside the support structure allows positioning of the coil in close proximity to the anatomical structure or tissue to be imaged, thereby providing an optimal SNR level and sensitivity. In some embodiments, the imaging system may have multiple X-nuclei coils that could be positioned inside the bra-shaped support structure. In such a case, X-nuclei coils can be mechanically or electrically altered between the imaging session depending on the targeted imaging nuclei, as discussed herein.

Despite the placement of X-nuclei coils inside the support system in close proximity to the anatomical structures or tissues to be imaged, the sensitivity may be limited due to the potential low dose of the targeted compound. In order to maximize the system sensitivity, in some embodiments, one or more passive flux focusing frequency selective elements may also be included in the system and housed within for example the cup-shaped support structures in close proximity to the X-nuclei coils in parallel to the coil. This passive flux focusing frequency selective element may comprise conductive wires or faces containing distributed capacitances and forming a closed current loop for enclosing for example a surface area on top of the anatomical structure or tissue to be imaged. As will be apparent to those of skill in the art, the distributed capacitance alongside with the inductance of the conductive wires or faces acts as a frequency selective element magnifying the X-nuclei signal and, therefore, the system's sensitivity by a confirmed factor of 2.5 (or higher), as discussed below.

Specifically, as discussed below, the second RF coil can be tuned to work with different labels; however, the signal amplification is caused by the flux-focusing element(s). Examples of simple configurations are provided herein and are discussed below for illustrative purposes.

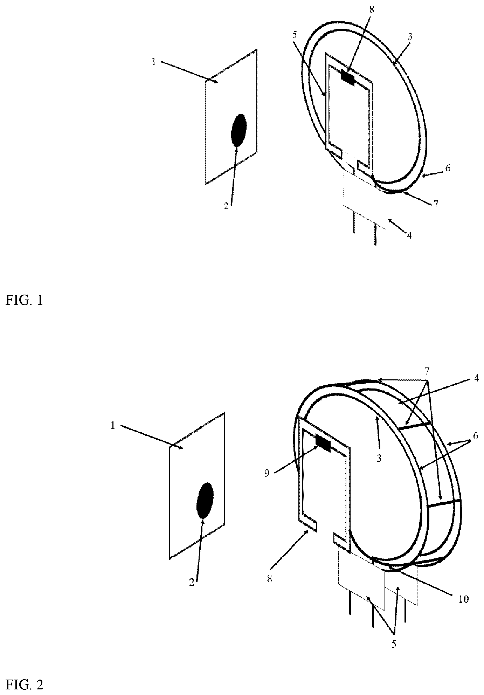

For example, Figure 1 shows one example of the imaging coil system with one multinuclear coil (3) and one passive frequency-selective flux focusing element (5). In this embodiment, the flux-focusing element is connected to the frame of the RF coil (6) via a semi-rigid arm (7) which allows precise positioning of it on top of the imaging object (2) within the region of interest (1). The flux-focusing element has at least one frequency-selective circuit (8). The RF coil has an impedance-matching circuit (4).

Furthermore, Figure 2 shows an example of the imaging coil system with two multinuclear coils (3,4) and one passive frequency selective flux-focusing element (8). Coils (3,4) are tuned to different frequencies and coil (4) can be tuned to a proton frequency for conventional MRI scans and better localization of the object (2) within the region of interest (1). RF coils are connected between their frames (6) using rigid connectors (7). The passive flux-focusing element has at least one frequency-selective circuit (9) and is connected to the frame of the RF coil (3) via a semi-rigid arm (10). Both RF coils have impedance-matching circuits (5).

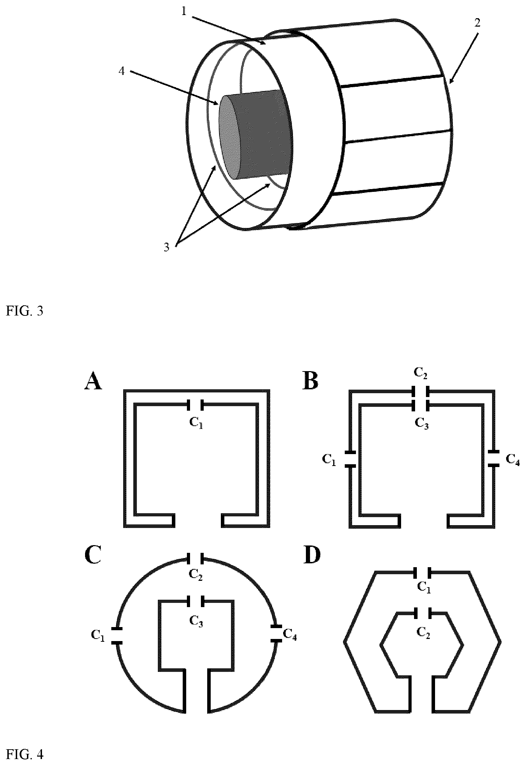

Yet further, Figure 3 shows an example of imaging coil system with one birdcage RF coil (2) and a passive frequency-selective flux focusing element (3) placed on the inner surface of the RF coil rigid dielectric frame (1). In this embodiment, the bird-cage coil (2) is located on the outer surface of the frame (1). The bird-cage coil can be either tuned to the frequency of the targeted X-nuclei or can be dual-tuned to the frequencies of the 1H (for better localization of the object (4)) and X-nuclei (for metabolic or functional imaging). This system can be used for example for either brain imaging or abdominal imaging.

The signal detection is performed by RF coil(s) (they are connected to the MRI scanner directly) which are used for imaging purposes. The signal amplification is performed by a passive (not connected to the MRI scanner) flux-focusing frequency-selective element(s), examples of which are shown in Figure 4, as discussed below. For example, these flux-focusing frequency-selective element(s) can be created utilizing the Lenz resonators concept (Figure 4). As would be appreciated by those of skill in the art, Lenz resonators comprise outer and inner conductive loops of any suitable geometry ( Fig. 4A, 4B - both are squares; Fig 4C - outer contour is circle, inner is square, Fig. 4D - both are hexagons) with some distributed capacitance on them. Due to the inductance of the conductive material, these circuits will have their own resonance frequency. If the incoming magnetic flux from the MR transmit coil has the same frequency as the resonance frequency of the Lenz resonator, the current will be induced in the resonator such that its magnetic field will add up to the incoming magnetic field resulting in the flux-focusing through the inner contour (due to the Lenz law). Moreover, the resonant properties of these circuits result in minimizing reactance of the Lenz resonators yielding to substantial amplification of the magnetic field through its inner loop at the resonance frequency. This will result in MR signal amplification during the imaging process. Lenz resonators have the substantial advantage of being frequency selective - they will amplify only the targeted signal without significant effect on the signal from the other coils and nuclei. Therefore, the Lenz resonators tuned to the X-nuclei frequency will not be affected by the conventional proton MRI image acquisition and vice versa.

Regarding Figure 4, this figure shows some possible examples of passive frequency-selective flux-focusing elements (Lenz resonators) that can be used as part of the invention. In these examples, the Lenz resonators comprise an external conductive loop and an internal conductive loop with some distributed capacitances on them. Capacitance values (C₁-C₄) are selected so as to tune the resonator to the frequency of the targeted nuclei. Due to the Lenz law, the incoming flux through the outer loop induces the electric current (if frequency of the incoming magnetic field matches the resonance frequency of the resonator), which creates an amplification and focusing of the magnetic flux through the inner loop. This flux amplification results in MRI signal amplification during the imaging.

The resonance frequency is determined by the capacitors included in the coil and flux-focusing element constructions. For example, C1-C4 on Fig. 4 will fully determine the resonance frequency of the Lenz resonator. The resonance frequency usually is permanent for the giving coil/resonator, due to the fixed values of the capacitors. It is possible to make the resonance frequency electronically adjustable by introducing variable capacitors in the schematic, however, variable capacitors usually have lower RF quality, resulting in poorer performance of the system overall. Therefore, although it is possible, creating a tunable system could be less desirable compared to creating a nucleus-specific detection system.

Furthermore, as will be appreciated by those of skill in the art, those capacitors can be any value, depending on the size of the element, type of the conductor used, and desired resonance frequencies.

In some embodiments, an array of the Lenz resonators can be used as a passive frequency selective flux-focusing element.

Regarding the arrangement of the coils, for example, in some embodiments, there may be a physical dielectric barrier in between them (Fig. 3), or their frames may be connected with each other (Fig. 1 and Fig. 2). Placement of the passive flux-focusing elements may also be either on the same dielectric barrier (Fig. 3) or by attaching them using a semi-rigid handle (Fig. 1 and 2). The first design will save space within the MR coil, whereas the semi-rigid handle will allow position adjustment of the element, resulting in better magnetic flux focusing and, therefore, better image/signal amplification.

For example, in some embodiments, the resonator is connected to the frame of the RF coil via a semi-rigid arm. In other embodiments, it is possible for some coil geometries, such as birdcage coil, to place the flux-focusing resonators on the inside surface of the coil frame. In this case, there is no mechanical connection needed, since the flux-focusing element is rigidly fixed to the coil frame.

The images may be taken either simultaneously or with some lag in between them. However, this should not affect the final performance of the system, only the overall scan time. Specifically, since RF coils are tuned to different frequencies, they can work simultaneously with no interference. The barrier is not necessary since each coil is manufactured inside an isolated frame and therefore, there should be no issues with putting them together. However, if no isolation case is used, then the dielectric barrier is needed between the coils.

As will be apparent to those of skill in the art, the imaging region is the same. In this case, we know for sure that everything is in the same location. The imaging system (essentially, a signal receiver) consists of multiple coils tuned to a different frequency. The underlying anatomical image can be acquired using an ¹H coil, mapping the proton distribution in the subject's tissues in the region of interest (ROI). Next (although could be done simultaneously), the signal from a different nucleus (for example, ¹⁹F) will be registered by a dedicated coil in the system tuned to that particular resonance frequency. This signal will be enhanced by the flux-focusing element (such as Lenz resonator) and converted into the image showing the location of the targeted molecule (or its metabolites). In the case of specific metabolic imaging, the received signal can be spectrally filtered to maintain only the signal from the metabolite of interest. Therefore, as the output, we will have an anatomical MRI image (shows the anatomy and acquired using ¹H) and a ¹⁹F image(s) showing the location of all molecular targets and metabolites. Since the imaging system did not move in between the scans, the images can be digitally superimposed on each other guaranteeing precise identification of the metabolites' location. Therefore, the key to the imaging is a) location and b) spectral resolution and sensitivity of the elements in the system.

In some embodiments, since the images are taken using the same system, the images will be self-aligned. Furthermore, the field-of-view for both images will be the same and fully confined within the imaging system, thereby guaranteeing perfect alignment of both images. Alternatively, in some embodiments, fiducials may be used on the skin for example by attaching a small capillary filled with an aqueous mixture of the imaging agent to the skin within the image field of view.

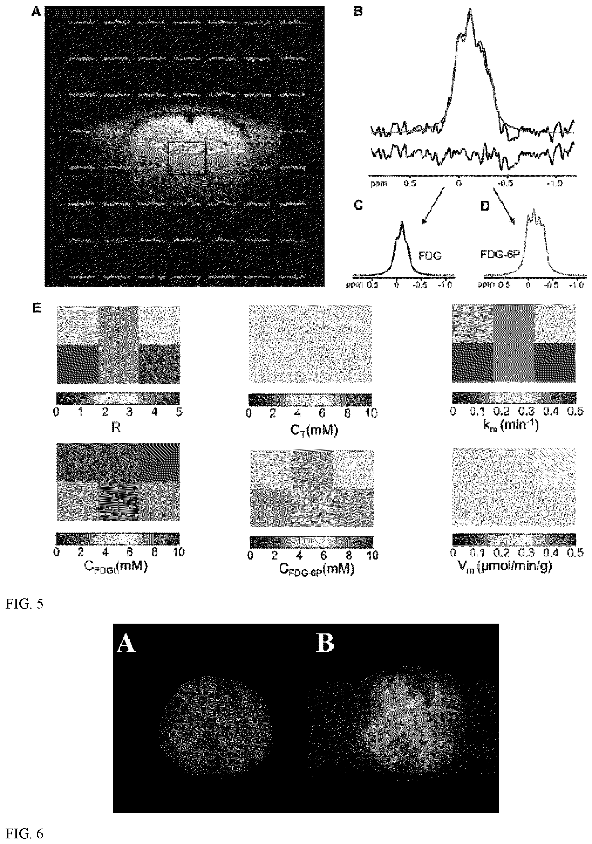

Figure 5 shows ¹⁹F signals (A-D) acquired from the rat brain during ¹⁹F-labled glucose infusion. (E) shows images of glucose and its metabolites (bottom raw) as well as their main kinetic parameters (top raw).

Once images are co-registered, we are able to see either the underlying metabolism of a targeted compound in different organs/tissues within the region of interest or drug uptake by the organ of interest. For example, the imaging agent and the metabolite that is produced may be detected, for example, at different frequencies and/or at different locations within a cell. As such, depending on the targeted compound selected, it is possible to learn about: a) the presence/absence of some specific lesions (such as tumors, etc.); b) the change in the organ function and diagnostics of the related diseases; c) efficacy of the treatment and/or need in treatment alternations; d) alternation in normal metabolism within the organs and diagnostics of the related diseases; e) evaluation of drug metabolism and biodistribution for optimization and personalization of treatment protocol. Currently, this is almost impossible to do with modern MRI imaging due to the overall low sensitivity, low signal-to-noise ratio of the targeted compounds in the conventional MRI, the need for realignment and co-registration of metabolic images and correspondent anatomical images, extremely long scan times, and susceptibility of these scans to the motion artifacts. Currently, any functional or metabolic images acquired with an MRI system are relatively low-resolution and with limited SNR. For example, ¹⁹F images shown in Fig. 6 were acquired by Coman D. et. al. (Coman D. et. al. Magn Reson Imag. 2014) using 7x7 spatial resolution with only 6 pixels corresponding to the region of interest. In order to achieve such low-resolution MR images, they had to utilize 11.7T magnetic field strength which is 4 times stronger than the clinically approved 3.0T field.

In contrast, the design of our system will allow maximization of the acquired signal due to the presence of the frequency selective flux focusing elements and due to the location of the receiver in close proximity to the organ of interest. Furthermore, our imaging system allows for precise positioning and focusing on the organ of interest, further maximizing the acquired signal and minimizing background noise.

An example of the signal amplification is shown in Figure 6 which shows images of a pomegranate acquired using (A) conventional quadrature birdcage proton coil and (B) our proposed imaging system with two passive flux-focusing Lenz resonators located inside the detection coil at 12 cm from a pomegranate. Specifically, Fig 6A shows the image of the pomegranate acquired using conventional clinical MRI system with quadrature birdcage proton coil. In contrast, the image shown in Fig 6B was acquired using our imaging system that contained two circular passive flux-focusing Lenz resonators located inside the birdcage coil at 12 cm distance from the pomegranate. As a result of this arrangement, the magnetic flux was focused into a circle with 7.5 cm diameter, thus covering almost the whole pomegranate. The detected signal-to-noise ratio was twice as large as compared to the SNR of a conventional MRI detection system, thus demonstrating at least two times better sensitivity with our design.

According to an aspect of the invention, there is provided a method of molecular imaging of a body region of an individual comprising:
administering a molecular imaging agent that contains one or more X-nuclei selected from the group consisting of ¹⁹F, ²³Na, ¹²⁹Xe, ³¹P and ¹³C, to an individual;
allowing the molecular imaging agent to distribute through the body of the individual, said imaging agent being metabolizable under certain conditions within the body of the individual;
acquiring an anatomical image of a body region of interest of the individual using a ¹H coil;
acquiring one or more X-nuclei image(s) of the molecular imaging agent and any metabolites of the molecular imaging agent within the body region of the individual using an X-nuclei coil specific for at least one of the X-nuclei in the molecular imaging agent;
superimposing the one or more X-nuclei image(s) of the body region of interest on the anatomical image of the body region of interest, thereby localizing metabolic activity and biodistribution of the molecular imaging agent within the body region of interest.

As will be appreciated by one of skill in the art, the number of X-nuclei images acquired will heavily depend on the specific type of the metabolic imaging agent used. For example, in some embodiments, a single image may include both the metabolic imaging agent and any metabolites generated therefrom.

As will be appreciated by one of skill in the art, the ¹H RF coil may be a stand-alone coil as discussed herein or may be part of a pre-existing imaging system, that is, an imaging system that the individual is placed into and then the X-nuclei RF coil is placed over the body region of interest, as discussed herein.

As will be appreciated by one of skill in the art, the molecular imaging agents may contain at least one X-nuclei incorporated into the molecular structure of the imaging agent. In these embodiments, the imaging agent may be for example chemotherapeutical or hormonal therapy agents, biosensors, or molecules whose metabolic activity is desired to be imaged.

In some embodiments of the invention, the chemotherapeutical and hormonal therapy agents may comprise ¹⁹F (such as for example but by no means limited to 5-fluorouracil, capecitabine, fulvestrant, abemaciclib) or ³¹P (such as for example but by no means limited to cyclophosphamide).

In some embodiments of the invention, molecular imaging biosensors may include ¹⁹F containing inorganic nanoparticles, liposomes, micelles, dendrimers, polymers, and other targeted ligands. In addition, biosensors may comprise supramolecular cages capable of incapsulating ¹²⁹Xe such as for example but by no means limited to cucurbit[6]uril, pillar[5]arene, and cryptophane-A.

In some embodiments of the invention, the molecular imaging agent is ¹⁹F-containing fluorodeoxyglucose (¹⁹F-FDG).

According to some aspects of the invention, one X-nuclei image and ¹H anatomical image can be acquired simultaneously.

According to some aspects of the invention, the system may be utilized to monitor and evaluate metabolic activities using ¹H MRI. In addition, the system may be utilized to monitor naturally occurring metabolism, that is, metabolism without additional interventions, in the body. As will be appreciated by one of skill in the art, the metabolism of phosphomonoester, choline, glucose, lactate, nucleotide triphosphates, inorganic phosphates, and phosphocreatine may be visualized using our novel imaging system.

In some embodiments, the imaging system may be utilized for molecular and metabolic imaging of chest and abdomen. In these embodiments, the imaging system comprises an ¹H MRI coil and at least one X-nuclei MRI coil.

In these embodiments, the MRI coils are positioned on either rigid or flexible support structures, for example, of a generally or substantially cylindrical shape. Support structure can open on one side to allow an individual to lie down inside the coil. The ¹H MRI coil consist of one coil array housed on the outer surface of the cylindrical support system. The ¹H MRI coil array may comprise for example either one, two, four, eight, sixteen, thirty-two, or sixty-four elements. In some embodiments, a bird-cage ¹H coil may be used.

In these embodiments, the X-nuclei coil comprises a single coil array and is attached to a cylindrical support structure via a semi-flexible arm that allows selective placement anywhere inside the support structure. This allows for the placement of the X-nuclei coil in close proximity to the region of interest, thereby maximizing the SNR and sensitivity.

In some embodiments, the X-nuclei coil can be housed on the inner surface of the support structure.

In use, the X-nuclei coil is tuned to a resonance frequency of nuclei of interest (¹⁹F, ³¹P, ¹²⁹Xe, ²³Na, ¹³C). In some embodiments, a passive flux focusing frequency selective element may also be included in the system and housed in front of the X-nuclei coil for example on a rigid thin plastic frame that is perpendicular to the direction of the alternating RF magnetic field produced by the coil. These passive flux-focusing RF elements have a similar design as described above and are tuned to the same resonance frequency as the X-nuclei coil.

In some embodiments, the ¹H MRI coil and X-nuclei MRI coil may be placed on opposite sides of a rigid support structure that has for example the shape of half of an elliptical cylinder which can have different sizes of semi-major and semi-minor axes. In such a design, the passive flux focusing frequency selective elements are placed in parallel to the X-nuclei coil.

In some embodiments, the imaging system may have multiple X-nuclei coils that are positioned inside a bra-shaped support structure. In these embodiments, the X-nuclei coils can be mechanically or electrically altered between imaging sessions depending on the targeted imaging nuclei.

According to an aspect of the invention, there is provided a method of molecular breast imaging of an individual comprising:
administering a molecular imaging agent that contains one or more X-nuclei selected from the group consisting of ¹⁹F, ²³Na, ¹²⁹Xe, ³¹P and ¹³C, into an individual;
allowing a molecular imaging agent to distribute through the body of the individual, said imaging agent being metabolizable under certain conditions within the body of the individual;
acquiring an anatomical breast image using a ¹H coil of at least a portion of the breast tissue of the individual;
acquiring one or more X-nuclei image(s) of the molecular imaging agent and any metabolites of the molecular imaging agent of the at least a portion of the breast tissue of the individual using an X-nuclei coil specific for at least one of the X-nuclei in the molecular imaging agent; and
superimposing the one or more X-nuclei image(s) on the anatomical breast image, thereby localizing metabolic activity and biodistribution of the molecular imaging agent within the at least a portion of the breast tissue of the individual.

As will be appreciated by one of skill in the art, the number of X-nuclei images acquired will heavily depend on the specific type of the metabolic imaging agent used. For example, in some embodiments, a single image may include both the metabolic imaging agent and any metabolites generated therefrom.

As will be appreciated by one of skill in the art, the ¹H RF coil may be a stand-alone coil as discussed herein or may be part of a pre-existing imaging system, that is, an imaging system that the individual is placed into and then the X-nuclei RF coil is placed over the body region of interest, as discussed herein.

According to another aspect of the invention, there is provided a method of molecular abdominal or chest imaging of an individual comprising:
injecting a molecular imaging agent that comprises one or more X-nuclei selected from the group consisting of ¹⁹F, ²³Na, ¹²⁹Xe, ³¹P and ¹³C, into the individual;
allowing the molecular imaging agent to distribute through the body of the individual, said molecular imaging agent being metabolizable in the body of the individual under certain conditions;
acquiring an anatomical abdominal or chest image using a ¹H RF coil;
acquiring one or more X-nuclei image(s) of the molecular imaging agent of a portion of the individual's body corresponding to the anatomical abdominal or chest image and any metabolites of the molecular imaging agent using an X-nuclei coil specific for at least one of the X-nuclei in the molecular imaging agent;
superimposing the one or more X-nuclei image(s) on the anatomical abdominal or chest image, thereby localizing metabolic activity and biodistribution of the molecular imaging agent in the abdomen or chest of the individual.

In some embodiments of the invention, the ¹H image is taken using an ¹H RF coil within a pre-existing MR imaging system and the individual is first placed into the imaging system, either after or before the molecular imaging agent is administered, for example, by injection of the molecular imaging agent.

As will be appreciated by one of skill in the art, the number of X-nuclei images acquired will heavily depend on the specific type of the metabolic imaging agent used. For example, in some embodiments, a single image may include both the metabolic imaging agent and any metabolites generated therefrom.

As will be appreciated by one of skill in the art, the ¹H RF coil may be a stand-alone coil as discussed herein or may be part of a pre-existing imaging system, that is, an imaging system that the individual is placed into and then the X-nuclei RF coil is placed over the body region of interest, as discussed herein.

As will be appreciated by one of skill in the art, the molecular imaging agents comprise at least one X-nuclei (¹⁹F, ³¹P, ¹²⁹Xe, ²³Na, ¹³C) incorporated in their molecular structure.

The molecular imaging agents may be chemotherapeutical agents, biosensors, or molecules whose metabolic activity is desired to be imaged.

In some embodiments of the invention, the chemotherapeutical and/or hormonal therapy agents may comprise ¹⁹F (such as for example but by no means limited to 5-fluorouracil, capecitabine, gemcitabine) or ³¹P (such as for example butby no means limited to cyclophosphamide and ifosfamide).

In some embodiments of the invention, the molecular imaging biosensors may include ¹⁹F or ³¹P containing inorganic nanoparticles, liposomes, micelles, dendrimers, and polymers. In addition, biosensors may comprise supramolecular cages capable of incapsulating ¹²⁹Xe including but not limited to cucurbit[6]uril, pillar[5]arene, and cryptophane-A.

In some embodiments of the invention, the molecular imaging agent is ¹⁹F-containing fluorodeoxyglucose (¹⁹F-FDG).

According to some aspects of the invention, one X-nuclei image and ¹H anatomical image are acquired simultaneously.

In some embodiments, the imaging system is used for functional imaging of the lungs. In these embodiments, the X-nuclei coil is tuned either to ¹⁹F or ¹²⁹Xe.

According to another aspect of the invention, there is provided an MRI imaging device comprising a ¹H MRI coil and at least one X-nuclei coil positioned on a support structure having a shape suitable for enclosing a body portion, for example, a tissue portion or an organ of interest, wherein the X-nuclei is selected from the group consisting of ¹⁹F, ²³Na, ¹²⁹Xe, ³¹P and ¹³C.

According to some aspects of the invention, the system may be utilized to monitor and evaluate metabolic activities using ¹H MRI. In addition, the system can be utilized to monitor metabolism naturally occurring in the body. As will be appreciated by one of skill in the art, the metabolism of phosphomonoester, choline, glucose, lactate, creatine, proline, nucleotide triphosphates, inorganic phosphates, and phosphocreatine can be visualized using the novel imaging system.

In some embodiments, the MRI imaging system can be utilized for molecular and metabolic imaging of the brain. In these embodiments, the imaging system comprises a ¹H MRI coil and at least one X-nuclei MRI coil.

In these embodiments, the MRI coils may be placed on the outer side (¹H MRI coil) and the inner side (X-nuclei coil) respectively of a rigid support structure that has for example either a cylindrical shape or shape of an elliptical cylinder. In these embodiments, the support structure may open on one side so that an individual can place their head inside the coil. The ¹H MRI may comprise one coil array housed on the outer surface of the cylindrical support system. The ¹H MRI coil array may comprise either one, two, four, eight, sixteen, thirty-two, or sixty-four elements. In some embodiments, a bird-cage ¹H coil may be used.

The X-nuclei coil comprises a single coil array and may be attached for example to a generally or substantially cylindrical support structure via a semi-flexible arm that allows selective placement of the X-nuclei coil anywhere inside the support structure. This allows for the placement of the X-nuclei coil in close proximity to the region of interest, thereby maximizing the SNR and sensitivity.

In some embodiments, the X-nuclei coil may be housed on the inner surface of the support structure.

The X-nuclei coil is tuned to a resonance frequency of nuclei of interest (¹⁹F, ³¹P, ¹²⁹Xe, ²³Na, ¹³C).

In some embodiments, a passive flux focusing frequency selective element may also be included in the system and housed or positioned or be configured such that it is in front of the X-nuclei coil, for example, on a rigid thin plastic frame that is perpendicular to the direction of the alternating RF magnetic field produced by the coil. These passive flux-focusing RF elements have a similar design as described above and are tuned to the same resonance frequency as the X-nuclei coil.

In some embodiments, the imaging system may have multiple X-nuclei coils that may be positioned inside the bra-shaped support structure. In these embodiments, X-nuclei coils may be mechanically or electrically altered between imaging session depending on the targeted imaging nuclei.

In some embodiments, the rigid support structure may have a shape of a helmet.

According to another aspect of the invention, there is provided a method of molecular brain imaging of an individual comprising:
injecting a molecular imaging agent that comprises one or more X-nuclei selected from the group consisting of ¹⁹F, ²³Na, ¹²⁹Xe, ³¹P and ¹³C, into the individual;
allowing the molecular imaging agent to distribute throughout the body, said molecular imaging agent being metabolizable in the body under certain conditions;
acquiring an anatomical brain image using a ¹H RF coil;
acquiring one or more X-nuclei image(s) of the molecular imaging agent and any metabolites of the molecular imaging agent using an X-nuclei coil specific for at least one of the X-nuclei in the molecular imaging agent; and
superimposing the one or more X-nuclei image(s) on the anatomical brain image, thereby localizing metabolic activity and biodistribution of the molecular imaging agent in the brain of the individual.

As will be appreciated by one of skill in the art, the number of X-nuclei images acquired will heavily depend on the specific type of the metabolic imaging agent used. For example, in some embodiments, a single image may include both the metabolic imaging agent and any metabolites generated therefrom.

As will be appreciated by one of skill in the art, the ¹H RF coil may be a stand-alone coil as discussed herein or may be part of a pre-existing imaging system, that is, an imaging system that the individual is placed into and then the X-nuclei RF coil is placed over the body region of interest, as discussed herein.

As will be appreciated by one of skill in the art, the molecular imaging agents may contain at least one X-nuclei (¹⁹F, ³¹P, ¹²⁹Xe, ²³Na, ¹³C) in their molecular structure.

The molecular imaging agents may be chemotherapeutical agents, biosensors, or molecules whose metabolic activity is desired to be imaged.

In some embodiments of the invention, the chemotherapeutical and hormonal therapy agents may contain ¹⁹F (such as for example but by no means limited to belzutifan, dabrafenib mesylate, and trametinib) or ³¹P (such as for example but by no means limited to cyclophosphamide).

In some embodiments of the invention, the molecular imaging biosensors may include ¹⁹F or ³¹P containing inorganic nanoparticles, liposomes, micelles, dendrimers, and polymers. In addition, biosensors may comprise supramolecular cages capable of incapsulating ¹²⁹Xe such as cucurbit[6]uril, pillar[5]arene, and cryptophane-A.

In some embodiments of the invention, the molecular imaging agent is ¹⁹F-containing fluorodeoxyglucose (¹⁹F-FDG).

According to some aspects of the invention, one X-nuclei image and ¹H anatomical image can be acquired simultaneously.

According to some aspects of the invention, the system is utilized to monitor and evaluate metabolic activities using ¹H MRI. In addition, the system may be utilized to monitor metabolism naturally occurring in the body. As will be appreciated by one of skill in the art, the metabolism of phosphomonoester, N-acetylaspartate, choline, glucose, lactate, creatine, proline, nucleotide triphosphates, glutamate/glutamine, inorganic phosphates, and phosphocreatine can be visualized using the imaging system.

In some embodiments, the system can be utilized to monitor oxygen metabolism of the brain.

As will be appreciated by one of skill in the art, in some embodiments, the system or parts thereof may be utilized to acquire MR images of superior SNR, resolution, and with a low scan time.

While the preferred embodiments of the invention have been described above, it will be recognized and understood that various modifications may be made therein, and the appended claims are intended to cover all such modifications which may fall within the spirit and scope of the invention.

## Claims

1. A method of molecular imaging of a body region of an individual comprising:
administering a molecular imaging agent that contains one or more X-nuclei selected from the group consisting of ¹⁹F, ²³Na, ¹²⁹Xe, ³¹P and ¹³C, to an individual;
allowing the molecular imaging agent to distribute through the body of the individual, said imaging agent being metabolizable under certain conditions within the body of the individual;
acquiring an anatomical image of a body region of interest of the individual using a ¹H coil;
acquiring one or more X-nuclei image(s) of the molecular imaging agent and any metabolites of the molecular imaging agent within the body region of the individual using an X-nuclei coil specific for at least one of the X-nuclei in the molecular imaging agent;
superimposing the one or more X-nuclei image(s) of the body region of interest on the anatomical image of the body region of interest, thereby localizing metabolic activity and biodistribution of the molecular imaging agent within the body region of interest.

2. The method according to claim 1 wherein the imaging agent is a chemotherapeutical therapy agent, a hormonal therapy agent, a biosensor, or a molecule whose metabolic activity is desired to be imaged.

3. The method according to claim 2 wherein the molecular imaging agent is a biosensor and the biosensor comprises a supramolecular cage.

4. The method according to claim 3 wherein the supramolecular cage comprises cucurbit[6]uril, pillar[5]arene, or cryptophane-A.

5. The method according to claim 1 wherein the molecular imaging agent is ¹⁹F-containing fluorodeoxyglucose (¹⁹F-FDG).

6. The method according to claim 1 wherein the X-nuclei image and the ¹H anatomical image are acquired simultaneously.

7. The method according to claim 1 wherein at least one of the MRI coils is positioned on a rigid or flexible support structures having a generally or substantially cylindrical shape.

8. The method according to claim 1 wherein the ¹H MRI coil is housed on an outer surface of the cylindrical support system and the X-nuclei coil is housed on an inner surface of the cylindrical support system.

9. The method according to claim 1 wherein the X-nuclei coil is configured with a passive flux focusing frequency selective element.

10. The method according to claim 9 wherein the passive flux frequency selective element is housed in front of the X-nuclei coil on a rigid thin plastic frame that is perpendicular to the direction of an alternating RF magnetic field produced by the X-nuclei coil.

11. The method according to claim 1 wherein the body region corresponds to an organ, a brain, a breast, a chest, or an abdomen.

12. The method according to claim 1 wherein the metabolic imaging agent is a chemotherapeutical therapy agent, a hormonal therapy agent, a biosensor, or a molecule whose metabolic activity is desired to be imaged.

13. An MRI imaging device comprising a ¹H MRI coil and at least one X-nuclei coil positioned on a support structure having a shape suitable for enclosing a body portion, for example, a tissue portion or an organ of interest, wherein the X-nuclei is selected from the group consisting of ¹⁹F, ²³Na, ¹²⁹Xe, ³¹P and ¹³C,.

14. The MRI imaging device according to claim 13 wherein the ¹H MRI coil is housed on an outer surface of the cylindrical support system and the X-nuclei coil is housed on an inner surface of the cylindrical support system.

15. The MRI imaging device according to claim 13 wherein the X-nuclei coil is configured with a passive flux focusing frequency selective element.

16. The MRI imaging device according to claim 13 wherein the passive flux frequency selective element is housed in front of the X-nuclei coil on a rigid thin plastic frame that is perpendicular to the direction of an alternating RF magnetic field produced by the X-nuclei coil.
